(19) Europäisches Patentamt
European Patent Office
Office européen des brevets

(11) **EP 3 236 858 B1**

(12) **EUROPEAN PATENT SPECIFICATION**

(45) Date of publication and mention
of the grant of the patent:
**09.09.2020  Bulletin 2020/37**

(21) Application number: **15816242.0**

(22) Date of filing: **07.12.2015**

(51) Int Cl.:
*A61B 8/08* (2006.01)          *A61B 8/00* (2006.01)
*A61B 5/02* (2006.01)          *A61B 5/021* (2006.01)
*A61B 8/06* (2006.01)

(86) International application number:
**PCT/IB2015/059391**

(87) International publication number:
**WO 2016/103087 (30.06.2016 Gazette 2016/26)**

(54) **A SYSTEM AND A METHOD FOR MEASURING ARTERIAL PARAMETERS**

SYSTEM UND VERFAHREN ZUR MESSUNG VON ARTERIENPARAMETERN

SYSTÈME ET PROCÉDÉ DE MESURE DE PARAMÈTRES ARTÉRIELS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **22.12.2014  IN 6458CH2014**

(43) Date of publication of application:
**01.11.2017  Bulletin 2017/44**

(73) Proprietor: **Koninklijke Philips N.V.
5656 AG Eindhoven (NL)**

(72) Inventors:
 • **PATIL, Ravindra Balasaheb**
   **5656 AE Eindhoven (NL)**
 • **PALANISAMY, Krishnamoorthy**
   **5656 AE Eindhoven (NL)**
 • **SISODIA, Rajendra Singh**
   **5656 AE Eindhoven (NL)**
 • **BUSSA, Nagaraju**
   **5656 AE Eindhoven (NL)**
 • **SETHURAMAN, Shriram**
   **5656 AE Eindhoven (NL)**
 • **BASAWARAJ PATIL OKALY, Vikram**
   **5656 AE Eindhoven (NL)**

(74) Representative: **Philips Intellectual Property &
Standards
High Tech Campus 5
5656 AE Eindhoven (NL)**

(56) References cited:
**WO-A2-2014/030174**

 • **TARDY Y ET AL: "Non-invasive estimate of the
mechanical properties of peripheral arteries from
ultrasonic and photoplethysmographic
measurements", CLINICAL PHYSICS AND
PHYSIOLOGICAL MEASUREMENT, INSTITUTE
OF PHYSICS PUBLISHING, BRISTOL, GB, vol. 12,
no. 1, 1 February 1991 (1991-02-01), pages 39-54,
XP020025922, ISSN: 0143-0815, DOI:
10.1088/0143-0815/12/1/003**

**EP 3 236 858 B1**

**Description**

**FIELD OF THE INVENTION**

The invention relates to measuring arterial parameters, more particularly to a system and a method for measuring arterial parameters using Radio Frequency Ultrasound.

**BACKGROUND OF THE INVENTION**

Generally, blood pressure measurement is considered to be an ideal biomarker to gauge the health condition of the patient. It is for this reason, blood pressure is measured prior to a surgery or during a normal health check-up and even when the patient is under medical observation. Conventionally, the blood pressure is measured on brachial artery to determine systolic and diastolic measures. These measures are usually taken as the standard for all the arteries.

Usually, the blood pressure is measured using a sphygmomanometer. The blood pressure measured herein is aortic blood pressure, from which the blood pressure on brachial artery is estimated with some variations. This approach essentially uses a cuff that is placed around an upper arm and inflated to supra-systolic pressure, following which the systolic and diastolic pressures are noted upon releasing the pressure caused due to inflation of the cuff. Here, it is important to use a cuff that is appropriately sized to suit the patient in order to reduce or eliminate deviations in blood pressure measurement.

On the other hand, blood pressure is also measured using ultrasound. Such ultrasound based approach for blood pressure measurement typically uses Brightness mode (B mode) imaging and image processing algorithm to track the movement of an artery.

Evidences show that the blood pressure measured on the brachial artery does not depict the actual blood pressure on the other peripheral arteries. It has become imperative to obtain absolute blood pressure on the specific artery to assess and treat vascular related disorders. Accordingly, localized blood pressure measurement along with flow assessment in the arteries greatly improves the ability to diagnose and monitor cardio-vascular disease.

In both the above described approaches, localized blood pressure measurement has not been realized. Besides this, measurement of various other arterial parameters such as Peak Systolic Velocity (PSV), Pulse Wave Velocity (PWV), arterial stiffness, etc. are not enabled through the known devices or techniques available.

US 2014143064 A1 discloses the use of blood velocity as a parameter to compute the blood pressure.

Certainly, there is a need for measuring localized blood pressure and other arterial parameters from a single measurement procedure. The invention is aimed at providing a solution for such need described herein above.

**OBJECTS OF THE INVENTION**

It is an object of the invention to provide a system for measuring localized arterial parameters;

It is another object of the invention to provide a system for measuring multiple localized arterial parameters including localized blood pressure, from a single measurement procedure;

It is yet another object of the invention to provide a method for measuring localized blood pressure by the system of the invention; and

Further object of the invention is to provide a method for measuring multiple other arterial parameters from a single measurement procedure by the system of the invention.

**SUMMARY OF THE INVENTION**

The invention is defined by the claims.

In one aspect of the invention, there is provided a system according to claim 1.

It is advantageous to measure localized blood pressure and other arterial parameters from a single measurement procedure leading to an improvement in the ability to diagnose and monitor cardio-vascular disease.

The invention provides a solution through a non-imaging and non-invasive system and method for measuring arterial parameters. Also, the invention provides a solution to measure the arterial parameters in a cuff-less way. Further, the invention eliminates the absolute need for PSV to determine the blood pressure.

In one preferred embodiment of the invention, a plurality of transducer elements are organized in a grid configuration so as to get localized RF ultrasound signals from multiple locations simultaneously from the said artery

In one preferred embodiment of the invention, RF ultrasound signal is used for determining the distension waveform, which captures the change in diameter of an artery caused due to pulsatile nature of blood, and of the pressure waveform, and of the localized blood pressure and other arterial parameters therefrom.

In another aspect of the invention, there is provided a method according to claim 10.

**BRIEF DESCRIPTION OF DRAWINGS:**

With reference to the accompanying drawings in which:

Fig. 1    shows the system for measuring the arterial parameters, in accordance with the invention;

Fig. 2    illustrates acquisition of RF data from an artery of interest;

Fig. 3 depicts the echo pattern obtained at different part of an artery of interest; and

Fig. 4 shows a sample distension waveform.

## DETAILED DESCRIPTION OF THE EMBODIMENTS

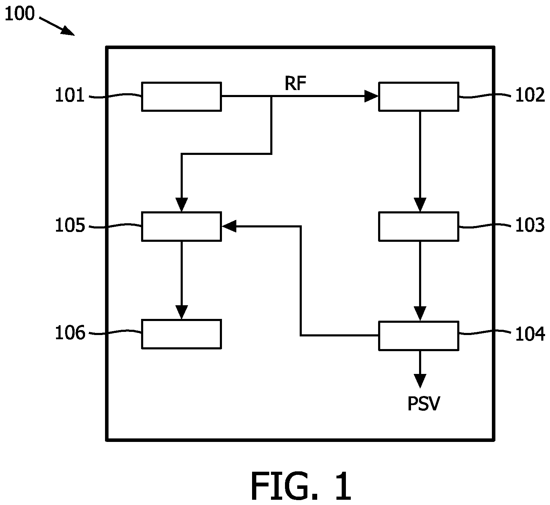

The invention is further described hereinafter with reference to Figs. 1 to 4 through non-exhaustive exemplary embodiments.

In Fig. 1, a system (100) for measuring the arterial parameters is shown. The system (100) comprises a Signal Unit (101) having a plurality of transducer elements organized in grid configuration. The transducer elements are operated either individually or collectively to provide raw RF ultrasound signals pertaining to the artery of interest subjected to measurement. The measurement in accordance with the invention includes utilizing the RF ultrasound signals both in raw form and demodulated form. The RF demodulation unit (102) demodulates the raw RF ultrasound signal. The detection unit (103) is provided to detect the presence of blood flow in the artery of interest using the Doppler Spectra data obtained from the demodulated RF ultrasound signal. Upon detecting the presence of blood flow, the artery of interest is specifically identified by the identification unit (104). A feedback signal is presented for continuous acquisition of data for few data cycle for further processing. The identification unit has one or more models pertaining to each of the corresponding arteries.

A processing unit (105) is provided to process the data purporting to the artery of interest, and explained herein after in the description.

The signal obtained from the transducer elements is affected with noise and transient behaviour. The raw RF echo signals are pre-processed to improve SNR, and the transients are removed by using matched filter followed by band pass filter. A non-linear gain is applied to suppress high amplitude reflections from skin gel interface.

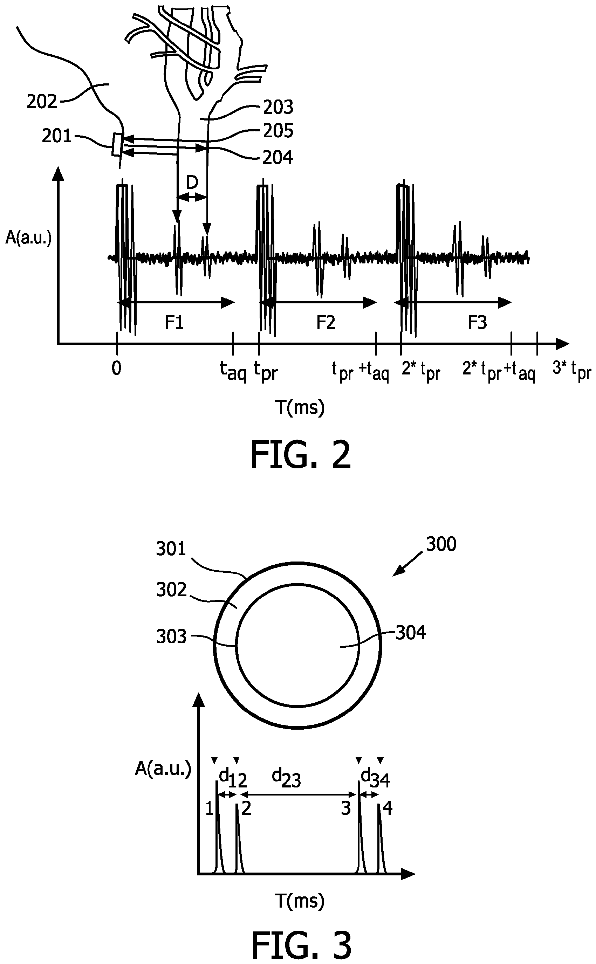

The signal frames are obtained over a particular interval of time as shown in Fig. 2, in respect of the artery of interest. The transducer (201) placed on the surface of the body sends ultrasound pulses into the body passing through the soft tissues (202) to reach the artery (203) like carotid artery. The ultrasound sound pulses (204) from the transducer is shown reaching the farther wall of the artery (203) and the same been reflected. The reflected ultrasound pulse is shown as (205). Apparently, the ultrasound pulses are transmitted and the same gets reflected from the near, as well as far walls of the artery whose diameter is D. The echoes are received by the same transducer. The data frame (F1, F2 and F3) containing amplitude information A (a, u) formed over time interval T(ms), of the reflected echoes are shown

The artery of interest, so identified is mapped to RF data for computation of artery specific distension waveform.

Fig. 3 shows the echo patterns (1, 2, 3, 4) after the ultrasound waves gets reflected from the different anatomical site such as adventitia (301), media (302) and intima (303) of the artery (300) of interest. The transition from the inner layer of the wall (303), intima, to the cavity inside the artery, lumen (304) produces a distinct echo, visible both at the near wall and the far wall. A stronger echo represents the outermost layer, the adventitia (301). The middle layer, media (302), is hypo-echoic.

Echo pattern obtained is used to compute the distension waveform as it could be observed that the echo's obtained from an artery over the different regions as shown in Fig. 3 is not uniform.

The far wall and the near wall of an artery can be separated by performing the echo gate tracking. The echoes are identified to find the region of interest in acquired signals where the anatomical structure of an artery could be located. To identify the region of interest from an echo pattern, a probabilistic approach such as Maximum Likelihood approach is used. Firstly, the energy plot for the given signal is obtained using sliding window approach and regions of maximum energy are identified and various features are extracted from these regions of interest for different patients across frames. The feature data is trained using a Gaussian mixture model by obtaining mean and covariance. On providing a test frame, features are extracted and tested against the model built to provide the likelihood of region of interests. The data are classified based on a single class approach such as wall class approach. The method uses a cut-off value of likelihood to assign points to wall class.

$$P\left(\frac{x}{C}\right) = \frac{1}{2\pi^{\frac{d}{2}}(\det(c))^{0.5}} e^{-(x-\mu)^T C^{-1}(x-\mu)}$$

where,

d- dimension of feature vector
c- covariance matrix
$\mu$- mean of Gaussian
x- feature vector for test point

Further, the variation of the blood flow during systolic and diastolic causes the change in the elastic property of an artery. This change in elastic property is manifested in diameter change of the artery. Based on the echoes obtained due to wall motion, successive signal frames are analyzed, and the distension waveform of the artery is computed as the difference between the near and far wall movements.

Considering the echoes obtained in two successive acquisitions, $NW_i(t)$ be the near wall echo and $FW_i(t)$ be the far wall echo in the $i^{th}$ acquisition. Now, the near and far wall echoes obtained in the next iteration may be expressed as

$$NW_{i+1}(t) = NW_i(t \pm \Gamma_{nw})$$

$$FW_{i+1}(t) = FW_i(t \pm \Gamma_{fw})$$

where $\Gamma_{fw}$ and $\Gamma_{nw}$ are the shifts in the near and far wall echoes, respectively. Echo tracking involves estimating these shifts and following the movement of the echoes accordingly. To estimate the time shift of the echoes in between successive acquisitions, a shift and search approach is employed, and is ideal to compute the maximum cross correlation between the signals $NW_i(t)$ and $NW_{i+1}(t)$ and estimating the shift $\Gamma_{fw}$ and $\Gamma_{nw}$ as the time corresponding to maximum of the cross correlation.

Once the delay is identified, the wall movements are computed based on the sound velocity (v)

$$d_{nw}(i) = 0.5 * v * [\Gamma_{nw}(i) + \Gamma_{nw}(i - 1)]$$

$$d_{fw}(i) = 0.5 * v * [\Gamma_{fw}(i) + \Gamma_{fw}(i - 1)]$$

The artery distension waveform is computed as the difference between the near wall and far wall movements and is given by

$$\Delta d(i) = d_{fw}(i) - d_{nw}(i)$$

Substituting t=(i/fprf), the distension waveform $\Delta d(t)$ can be determined as follows

$$\Delta d(t) = d_{fw}(t) - d_{nw}(t)$$

From the above mentioned distension waveform, peak to peak distension can be computed, which can be used to measure other arterial compliance measure.

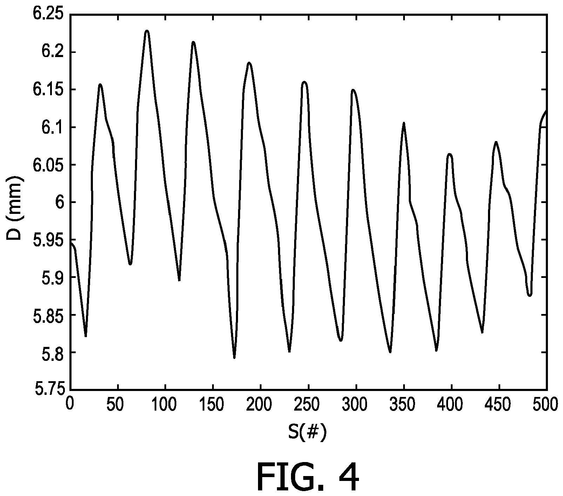

Fig. 4 shows the sample distension waveform representing the change in wall diameter D(mm) through successive frames over the Sample S(#).

The pressure change in an artery is manifested better, by the change in cross section of an artery. Arterial wall cross-section as function of time is further computed based on distension waveform by the following equation.

$$A(t) = \frac{\pi d(t)^2}{4} \qquad \text{eq (1)}$$

The functional relationship between the blood pressure waveform p(t) and arterial wall cross section A(t) is established as follows

$$p(t) = p_o e^{\gamma A(t)} \qquad \text{eq (2)}$$

Where $p_o$ is constant and $\gamma$ varies between arteries of a patient and across patients. A look up table and arterial model is required to measure respective $\gamma$ for the artery of interest. Identification Unit (104) as shown in Fig. 1 provides this input to estimate artery and artery specific $\gamma$ value for better accuracy of pressure waveform estimation.

From eq (2), the pressure wave form can be computed and systolic, diastolic and mean arterial pressure could be estimated. It is hereby possible to continuously monitor the blood pressure for an artery of interest in non-invasive and non-imaging based approach.

As the change in diameter and the pressure associated with artery of interest are available, other arterial compliance measures such as *Elastic modulus, Arterial distensibility, Arterial compliance and Stiffness index* can be computed as below, where $P_s$ is systolic and $P_d$ is diastolic pressure.

Elastic modulus,

$$E = \frac{\Delta P \, X \, D}{\Delta D}$$

Arterial Distensibility,

$$D = \frac{\Delta D}{\Delta P X D_d}$$

Arterial Compliance,

$$C = \frac{\Delta D}{\Delta P}$$

Stiffness index,

$$\varphi = \frac{\ln(P_s/P_d)}{(\frac{D_s - D_d}{D_d})}$$

The invention therefore provides continuous measurement of localized blood pressure of the artery of interest along with other arterial parameters and arterial compliance measures.

Only certain features of the invention have been specifically illustrated and described herein, and many modifications and changes will occur to those skilled in the art. The invention is not restricted by the preferred embodiment described herein in the description. It is to be noted that the invention is explained by way of exemplary embodiment and is neither exhaustive nor limiting. Certain aspects of the invention that not been elaborated herein in the description are well understood by one skilled in the art. Also, the terms relating to singular form used herein in the description also include its plurality and vice versa, wherever applicable. Any relevant modification or variation, which is not described specifically in the specification are in fact to be construed of being well within the scope of the invention. The presently disclosed embodiments are therefore considered in all respects to be illustrative and not restricted. The scope of the invention is indicated by the appended claims rather than the foregoing description and all changes that come within the meaning and range and equivalence thereof are intended to be embraced therein.

**Claims**

1. A system (100) for measuring arterial parameters using ultrasound, the system comprising:

   a signal unit (101) for providing a radio frequency (RF) ultrasound signal;
   a demodulator (102) for providing a demodulated radio frequency ultrasound signal;
   a detection unit (103) for detecting the presence of blood flow in an artery based on the demodulated radio frequency ultrasound signal;
   an identification unit (104) for identifying the artery based on a signal from the detection unit (103);
   a processing unit (105) for processing the radio frequency (RF) ultrasound signal and a signal from the identification unit (104) and for providing a distension waveform of the artery; and
   an estimating unit (106) for estimating at least one of a plurality of localized arterial parameters of the artery.

2. The system as claimed in claim 1, wherein the signal unit comprises a plurality of transducer elements organized in a grid configuration.

3. The system as claimed in claim 2, wherein the plurality of transducer elements are operated individually or collectively to provide RF ultrasound signal pertaining to an artery.

4. The system as claimed in claim 1, wherein the processing unit is provided for determining the motion of the vessel wall of the artery and the change in diameter of the artery.

5. The system as claimed in claim 1, wherein the processing unit is provided for obtaining a pressure waveform from the distension waveform of the artery.

6. The system as claimed in claim 5, wherein the processing unit is provided for mapping the distension waveform of the artery, with the pressure waveform of the artery identified, using the model of the artery.

7. The system as claimed in claim 1, wherein the estimation unit is provided for estimating at least one of the localized arterial parameters such as localized blood pressure, Peak Systolic Velocity (PSV), Pulse Wave Value (PWV), arterial compliance measures.

8. The system as claimed in claim 7, wherein the arterial compliance measures include elastic modulus, arterial distensibility, arterial compliance, stiffness index.

9. The system as claimed in any one of the preceding claims, wherein the system is a non-imaging and non-invasive based system for measuring arterial parameters.

10. A method for measuring arterial parameters using ultrasound, the method comprising:

    providing, by a signal unit (101), a radio frequency (RF) ultrasound signal;
    providing, by a demodulator (102), a demodulated radio frequency ultrasound signal;
    detecting the presence of blood flow in an artery by a detection unit (103) based on the demodulated radio frequency ultrasound signal;
    identifying the artery by an identification unit (104) based on a signal from the detection unit (103);
    processing, by a processing unit (105), the radio frequency (RF) ultrasound signal and a signal from the identification unit (104) and providing a distension waveform of the artery; and
    estimating, by an estimating unit (106), at least one of a plurality of localized arterial parameters of the artery.

11. The method as claimed in claim 10, wherein the processing comprises determining the motion of the vessel wall of the artery and the change in diameter of the artery.

12. The method as claimed in claim 10, wherein the processing comprises obtaining a pressure waveform from the distension waveform of the artery.

13. The method as claimed in claim 12 wherein the processing comprises mapping the distension waveform of the artery, with the pressure waveform of the artery identified, using the model of the artery.

14. The method as claimed in any one of the preceding claims 10 to 13, wherein the method is a non-imaging and non-invasive and continuous method for estimating localized arterial parameters such as localized blood pressure, Peak Systolic Velocity (PSV), Pulse Wave Value (PWV) and arterial compliance measures including elastic modulus, arterial distensibility, arterial compliance, stiffness index.

15. A computer program product comprising code means for performing the method as claimed in claims 10 to 14 when executed on a computer processor.

**Patentansprüche**

1. System (100) zum Messen von Arterienparametern unter Verwendung von Ultraschall, wobei das System umfasst:

   eine Signaleinheit (101) zum Bereitstellen eines Hochfrequenz(HF)-Ultraschallsignals;
   einen Demodulator (102) zum Bereitstellen eines demodulierten Hochfrequenz-Ultraschallsignals;
   eine Erfassungseinheit (103) zum Erfassen des Vorhandenseins eines Blutflusses in einer Arterie basierend auf dem demodulierten Hochfrequenz-Ultraschallsignal;
   eine Identifikationseinheit (104) zum Identifizieren der Arterie basierend auf einem Signal von der Detektionseinheit (103);
   eine Verarbeitungseinheit (105) zum Verarbeiten des Hochfrequenz(HF)-Ultraschallsignals und eines Signals von der Identifikationseinheit (104) und zum Bereitstellen einer Dehnungswellenform der Arterie; und
   eine Schätzeinheit (106) zum Schätzen mindestens eines von mehreren lokalisierten Arterienparametern der Arterie.

2. System nach Anspruch 1, wobei die Signaleinheit mehrere Wandlerelemente umfasst, die in einer Gitterkonfiguration organisiert sind.

3. System nach Anspruch 2, wobei die mehreren Wandlerelemente einzeln oder gemeinsam betrieben werden, um ein HF-Ultraschallsignal bereitzustellen, das eine Arterie betrifft.

4. System nach Anspruch 1, wobei die Verarbeitungseinheit zum Bestimmen der Bewegung der Gefäßwand der Arterie und der Änderung des Durchmessers der Arterie vorgesehen ist.

5. System nach Anspruch 1, wobei die Verarbeitungseinheit zum Erhalten einer Druckwellenform aus der Dehnungswellenform der Arterie vorgesehen ist.

6. System nach Anspruch 5, wobei die Verarbeitungseinheit zum Abbilden der Dehnungswellenform der Arterie vorgesehen ist, wobei die Druckwellenform der Arterie unter Verwendung des Modells der Arterie identifiziert wird.

7. System nach Anspruch 1, wobei die Schätzeinheit zum Schätzen mindestens eines der lokalisierten Arterienparameter wie lokalisierter Blutdruck, systolische Spitzengeschwindigkeit (PSV), Pulswellenwert (PWV), Arteriencompliance-Maßnahmen vorgesehen ist.

8. System nach Anspruch 7, wobei die Arteriencompliance-Maßnahmen den Elastizitätsmodul, die Arterienendehnbarkeit, die Arteriencompliance und den Steifheitsindex umfassen.

9. System nach einem der vorhergehenden Ansprüche, wobei das System ein nicht bildgebendes und nicht invasives basiertes System zum Messen von Arterienparametern ist.

10. Verfahren zum Messen von Arterienparametern unter Verwendung von Ultraschall, wobei das Verfahren umfasst:

    Bereitstellen eines Hochfrequenz(HF)-Ultraschallsignals durch eine Signaleinheit (101);
    Bereitstellen eines demodulierten Hochfrequenz-Ultraschallsignals durch einen Demodulator (102);
    Erfassen des Vorhandenseins eines Blutflusses in einer Arterie durch eine Erfassungseinheit (103) basierend auf dem demodulierten Hochfrequenz-Ultraschallsignal;
    Identifizieren der Arterie durch eine Identifikationseinheit (104) basierend auf einem Signal von der Detektionseinheit (103);
    Verarbeiten des Hochfrequenz-Ultraschallsignals (HF) und eines Signals von der Identifikationseinheit (104) durch eine Verarbeitungseinheit (105) und Bereitstellen einer Dehnungswellenform der Arterie; und
    Schätzen durch eine Schätzeinheit (106) mindestens eines von mehreren lokalisierten Arterienparametern der Arterie.

11. Verfahren nach Anspruch 10, wobei die Verarbeitung das Bestimmen der Bewegung der Gefäßwand der Arterie und der Änderung des Durchmessers der Arterie umfasst.

12. Verfahren nach Anspruch 10, wobei die Verarbeitung das Erhalten einer Druckwellenform aus der Dehnungswellenform der Arterie umfasst.

13. Verfahren nach Anspruch 12, wobei die Verarbeitung die Abbildung der Dehnungswellenform der Arterie umfasst, wobei die Druckwellenform der Arterie unter Verwendung des Modells der Arterie identifiziert wird.

**14.** Verfahren nach einem der vorhergehenden Ansprüche 10 bis 13, wobei das Verfahren ein nicht bildgebendes und nicht invasives und kontinuierliches Verfahren zum Schätzen von lokalisierten Arterienparametern wie lokalisierter Blutdruck, systolische Spitzengeschwindigkeit (PSV), Pulswellenwert (PWV) und Arteriencompliance-Maßnahmen ist, einschließlich Elastizitätsmodul, Arteriendehnbarkeit, Arteriencompliance, Steifheitsindex.

**15.** Computerprogrammprodukt, umfassend Codemittel zum Durchführen des Verfahrens gemäß den Ansprüchen 10 bis 14, wenn es auf einem Computerprozessor ausgeführt wird.


**Revendications**

**1.** Système (100) pour mesurer des paramètres artériels à l'aide d'ultrasons, le système comprenant:

une unité de signal (101) pour fournir un signal ultrasonore en radiofréquence (RF);
un démodulateur (102) pour fournir un signal ultrasonore en radiofréquence démodulé;
une unité de détection (103) pour détecter la présence de flux sanguin dans une artère sur la base du signal ultrasonore en radiofréquence démodulé;
une unité d'identification (104) pour identifier l'artère sur la base d'un signal provenant de l'unité de détection (103);
une unité de traitement (105) pour traiter le signal ultrasonore en radiofréquence (RF) et un signal provenant de l'unité d'identification (104) et pour fournir une forme d'onde de la distension de l'artère; et
une unité d'estimation (106) pour estimer au moins l'un d'une pluralité de paramètres artériels localisés de l'artère.

**2.** Système selon la revendication 1, dans lequel l'unité de signal comprend une pluralité d'éléments transducteurs organisés dans une configuration de grille.

**3.** Système selon la revendication 2, dans lequel les plusieurs éléments transducteurs sont actionnés individuellement ou collectivement pour fournir un signal ultrasonore RF relatif à une artère.

**4.** Système selon la revendication 1, dans lequel l'unité de traitement est prévue pour déterminer le mouvement de la paroi vasculaire de l'artère et le changement de diamètre de l'artère.

**5.** Système selon la revendication 1, dans lequel l'unité de traitement est prévue pour obtenir une forme d'onde de pression à partir de la forme d'onde de la distension de l'artère.

**6.** Système selon la revendication 5, dans lequel l'unité de traitement est prévue pour cartographier la forme d'onde de la distension de l'artère, avec la forme d'onde de la pression de l'artère identifiée, en utilisant le modèle de l'artère.

**7.** Système selon la revendication 1, dans lequel l'unité d'estimation est prévue pour estimer au moins l'un des paramètres artériels localisés tels que la pression sanguine localisée, la vitesse systolique maximale (PSV), la valeur d'onde de pouls (PWV), les mesures de la compliance artérielle.

**8.** Système selon la revendication 7, dans lequel les mesures de la compliance artérielle comprennent le module élastique, la distensibilité artérielle, la compliance artérielle, l'indice de rigidité.

**9.** Système selon l'une quelconque des revendications précédentes, dans lequel le système est un système de non-imagerie et non invasif pour mesurer des paramètres artériels.

**10.** Procédé de mesure de paramètres artériels utilisant des ultrasons, le procédé comprenant:

fournir, par une unité de signal (101), un signal ultrasonore en radiofréquence (RF);
fournir, par un démodulateur (102), un signal ultrasonore en radiofréquence démodulé;
détecter la présence de flux sanguin dans une artère par une unité de détection (103) sur la base du signal ultrasonore en radiofréquence démodulé;
identifier l'artère par une unité d'identification (104) sur la base d'un signal provenant de l'unité de détection (103);
traiter, par une unité de traitement (105), le signal ultrasonore en radiofréquence (RF) et un signal provenant de l'unité d'identification (104) et fournir une forme d'onde de la distension de l'artère; et
estimer, par une unité d'estimation (106), au moins l'un d'une pluralité de paramètres artériels localisés de l'artère.

**11.** Procédé selon la revendication 10, dans lequel le traitement comprend la détermination du mouvement de la paroi vasculaire de l'artère et du changement de diamètre de l'artère.

**12.** Procédé selon la revendication 10, dans lequel le traitement comprend l'obtention d'une forme d'onde de la pression à partir de la forme d'onde de la distension de l'artère.

**13.** Procédé selon la revendication 12, dans lequel le traitement comprend la cartographie de la forme d'onde de la distension de l'artère, avec la forme d'onde de la pression de l'artère identifiée, en utilisant le modèle de l'artère.

**14.** Procédé selon l'une quelconque des revendications 10 à 13 précédentes, dans lequel le procédé est un procédé de non-imagerie et non invasif et continu pour estimer des paramètres artériels localisés tels que la pression sanguine localisée, la vitesse systolique maximale (PSV), la valeur de l'onde de pouls (PWV) et les mesures de la compliance artérielle, y compris le module élastique, la distensibilité artérielle, la compliance artérielle, l'indice de rigidité.

**15.** Produit de programme informatique comprenant des moyens de code pour exécuter le procédé selon les revendications 10 à 14 lorsqu'il est exécuté sur un processeur informatique.

FIG. 1

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014143064 A1